# EUROPÄISCHE PATENTSCHRIFT

(11) **EP 1 469 928 B1**
(45) Veröffentlichungstag und Bekanntmachung des Hinweises auf die Patenterteilung: **22.11.2006**
(21) Anmeldenummer: 03704367.6
(22) Anmeldetag: 09.01.2003
(51) Int. Cl.: B01D 19/00, A61M 1/02

(54) **STERILES SYSTEM UND VERFAHREN ZUM FILTRIEREN VON BIOLOGISCHEN ODER MEDIZINISCHEN FLÜSSIGKEITEN, INSBESONDERE VON BLUT- ODER BLUTKOMPONENTEN**
STERILE SYSTEM AND METHOD FOR FILTERING BIOLOGICAL OR MEDICAL LIQUIDS, ESPECIALLY BLOOD OR BLOOD CONSTITUENTS
SYSTEME STERILE ET PROCEDE POUR FILTRER DES LIQUIDES BIOLOGIQUES OU MEDICAUX, NOTAMMENT DU SANG OU DES COMPOSANTS SANGUINS

(30) Priorität: 29.01.2002 IT TO20020080
(43) Veröffentlichungstag der Anmeldung: 27.10.2004
(73) Patentinhaber: Fresenius Hemocare Italia S.r.l., I-41032 Cavezzo, (Modena) (IT)
(72) Erfinder: VERRI, Paolo, I- 41033 Concordia (IT); MARI, Giorgio, I-41037 Mirandola (IT)
(74) Vertreter: Luderschmidt, Schüler & Partner
(86) Internationale Anmeldenummer: PCT/EP2003/000127
(87) Internationale Veröffentlichungsnummer: WO 2003/064000

(56) Entgegenhaltungen:
- EP-A- 0 888 789
- DE-A- 19 733 407
- US-A- 5 601 730

## Beschreibung

Die Erfindung betrifft ein steriles System zum Filtrieren von biologischen oder medizinischen Flüssigkeiten, insbesondere von Blut oder Blutkomponenten, sowie ein Verfahren zum Filtrieren von biologischen oder medizinischen Flüssigkeiten.

Aus der EP 0 642 801 A1 ist eine Filtrationsvorrichtung bekannt, die einen Sammelbeutel und ein Schlauchleitungssystem mit einem Filter umfasst. Der Beutel, der die zu filtrierende Flüssigkeit enthält, ist nicht Bestandteil der Vorrichtung. Das Schlauchleitungssystem der bekannten Filtriervorrichtung weist neben der Schlauchleitung, in die der Filter geschaltet ist, noch eine Bypassleitung mit einem Rückschlagventil auf, die stromauf bzw. stromab des Filters von der Schlauchleitung abzweigt, so dass nach Beendigung des Filträtionsvorganges Luft aus dem Sammelbeutel in den die zu filtrierende Flüssigkeit enthaltenden Beutel strömen kann. Die ausgangsseitige Kammer des Filters weist eine zusätzliche Belüftungsöffnung auf, die über eine Schlauchleitung mit einer Tropfkammer verbunden ist, die in den zum Filter führenden Schlauchleitungsabschnitt geschaltet ist.

Ein Filtriersystem für biologische oder medizinische Flüssigkeiten ist auch aus der WO 91/17809 bekannt. Die zu filtrierende Flüssigkeit wird unter dem Einfluss der Schwerkraft aus einem Vorlagebeutel über eine Schlauchleitung und einen Filter in einen Sammelbeutel überführt. Nachteilig ist, dass nach der Beendigung des Filtrationsvorganges, d. h. nachdem die Flüssigkeit aufgehört hat in den Sammelbeutel zu fliessen, noch Flüssigkeit in der Schlauchleitung und dem Filter verbleibt. Zur Rückgewinnung dieser Flüssigkeit ist an der Schlauchleitung stromauf des Filters ein Gaseinlass und stromab des Filters ein Gasauslass vorgesehen. Nach der Beendigung des Filtrationsvorganges wird in das System über den Einlass Gas geleitet, das unter Verdrängung der in dem System befindlichen Flüssigkeit wieder über den Auslass abgeführt wird.

Bei einer bevorzugten Ausführungsform des aus der WO 91/17809 bekannten Filtriersystems wird vorgeschlagen, zur vollständigen Entleerung des Systems eine -auslassleitung stromauf des Filters und eine Entlüftungsleitung stromab des Filters an der Schlauchleitung vorzusehen. Belüftungs- und Entlüftungsleitung führen in einen gemeinsamen Be- bzw. Entlüftungsbeutel, wobei hydrophobe Membranen an beiden Leitungen verhindern, dass Flüssigkeit in den Behälter strömt. Das Filtriersystem beruht auf dem Prinzip, dass zur Belüftung des Systems Luft aus dem Behälter über die -auslassleitung dem System zugeführt wird und zur Entlüftung des Systems Luft aus dem System über die Entlüftungsleitung dem Behälter zugeführt wird. Daher werden die Anschlussstellen auch als Gasein- und -auslass bezeichnet.

Die EP 0 888 789 A1 beschreibt ein steriles System zum Filtrieren von Blut oder Blutkomponenten, die ebenfalls von einem kollabierbaren Behältnis Gebrauch macht, in dem die im System befindliche Luft vor der Einleitung des Filtrationsvorganges gesammelt und nach Beendigung des Filtrationsvorganges zur vollständigen Entleerung des Systems benutzt wird. Der in die Sammelleitung geschaltete Filter des bekannten Filtriersystems weist zwei Kammern auf, von denen die erste Kammer über eine erste Schlauchleitung und die zweite Kammer über eine zweite Schlauchleitung mit dem kollabierbaren Behältnis verbunden ist. Beide Schlauchleitungen sind mit einer hydrophoben Membran verschlossen. Die zu filtrierende Flüssigkeit fliesst in das Sammelbehältnis unter Verdrängung der in den beiden Filterkammern befindlichen Luft durch beide Schlauchleitungen in das kollabierbare Behältnis. Zur vollständigen Entleerung des Systems wird zunächst die zweite Schlauchleitung geschlossen, wobei Luft aus dem kollabierbaren Behältnis über die erste Schlauchleitung in die erste Kammer des Filters strömt. Daraufhin wird die zweite Schlauchleitung wieder geöffnet, so dass Luft über die zweite Schlauchleitung in die zweite Kammer des Filters strömt. Dadurch wird erreicht, dass zuerst die erste und dann die zweite Filterkammer belüftet wird.

Das bekannte Filtriersystem hat sich in der Praxis bewährt. Als nachteilig erweist sich jedoch die Verwendung von zwei Sterilfiltern, die den Herstellungsaufwand vergrössem.

Der Erfindung liegt daher die Aufgabe zugrunde, ein einfach zu handhabendes und einfach herzustellendes System zum Filtrieren von biologischen oder medizinischen Flüssigkeiten, insbesondere von Blut oder Blutkomponenten zu schaffen, dass sich vollständig entleeren lässt, ohne dass Luft von aussen dem System zugeführt werden müsste, wodurch die Sterilität des Systems verloren ginge. Eine weitere Aufgabe der Erfindung ist, ein mit einfachen Mitteln durchzuführendes Verfahren zum Filtrieren von biologischen oder medizinischen Flüssigkeiten anzugeben, dass die vollständige Überführung der Flüssigkeit aus einem Behältnis in ein anderes Behältnis über einen Filter unter sterilen Bedingungen erlaubt.

Die Lösung dieser Aufgabe erfolgt erfindungsgemäss mit den in den Patentansprüchen 1 und 7 angegebenen Merkmalen.

Bei dem erfindungsgemässen Filtriersystem bzw. Verfahren wird die bereits im System befindliche sterile Luft vor der Einleitung des Filtrationsvorganges, d. h. bevor die Strömungsverbindung zu dem Sammelbehältnis freigegeben wird, gesammelt und nach Beendigung des Filtrationsvorganges, d. h. wenn die Flüssigkeit aufgehört hat, in das Sammelbehältnis zu strömen, zur vollständigen Entleerung des Systems benutzt.

Das erfindungsgemässe Filtriersystem zeichnet sich dadurch aus, dass nur eine der beiden Lufteinlass- bzw. -auslassleitungen mit einem Rückschlagventil oder einer hydrophoben Membran verschlossen ist. Während die Flüssigkeit über die Sammelleitung in das Sammelbehältnis fliesst, wird der zweite Luftein- bzw. - auslass des kollabierbaren Behältnisses verschlossen. Erst zur vollständigen Entleerung des Systems wird der zweite Luftein- bzw. -auslass geöffnet. Die in dem kollabierbaren Behältnis befindliche Luft strömt einerseits über die erste Luftein- bzw. -auslassleitung in die erste Kammer des Filters, wobei das Rückschlagventil oder die hydrophobe Membran einen Durchlass für die Luft freigibt, und andererseits aus dem kollabierbaren Behältnis über die zweite Lufteinlass- bzw. -auslassleitung in die zweite Filterkammer, so daß auch die zweite Kammer entleert wird.

Unter einer Luftein- bzw. -auslassleitung werden alle Mittel verstanden, mit denen Luft dem kollabierbaren Behältnis zugeführt oder Luft aus dem Behältnis abgeführt werden kann. Die Luftein- bzw. -auslassleitung kann auch eine Öffnung in dem Behältnis oder ein an das Behältnis angeformtes Anschlussstück oder Schlauchleitungsstück umfassen, d. h. das Rückschlagventil oder die hydrohobe Membran kann auch in das kollabierbare Behältnis integriert sein.

Unter einer die Luftein- bzw. -auslassleitung verschliessenden hydrophoben Membran werden alle Mittel verstanden, die eine Zufuhr von Luft in die bzw. aus der Luftein- bzw. -auslassleitung erlauben, eine Flüssigkeitsströmung jedoch verhindern. Die hydrophobe Membran kann innerhalb oder ausserhalb des kollabierbaren Behältnisses angeordnet sein, beispielsweise kann die hydrophobe Membran in einem zu dem Luftein- bzw. -auslass führenden Schlauchleitungsstück angeordnet sein. Ein Rückschlagventil anstelle einer hydrophoben Membran als alternative Ausführungsform erlaubt einen Durchlass von Fluid nur in Richtung der ersten Filterkammer, nicht aber in das kollabierbare Behältnis.

Eine besonders schnelle vollständige Entleerung des System wird erreicht, wenn die zweite Lufteinlass- oder -auslassleitung an einer dem Auslass der zweiten Filerkammer gegenüberliegenden Seite in die zweite Kammer mündet, da dann gleichzeitig Luft aus dem kollabierbaren Behältnis von oben in die zweite Filterkammer strömen und Flüssigkeit von unten aus der zweiten Kammer abfließen kann.

Die erste Lufteinlass- bzw. -auslassleitung kann von der ersten Sammelleitung abzweigen oder direkt an der ersten Filterkammer angeschlossen sein.

Das kollabierbare Behältnis ist vorzugsweise als flexibler Kunststoffbeutel ausgebildet, der mit den Lufteinlass- bzw. -auslassleitungen verschweisst ist. Zur Befestigung kann der Kunststoffbeutel mit einer Lasche versehen sein, durch die sich die erste Sammelleitung erstreckt. Es ist aber auch möglich, dass das kollabierbare Behältnis direkt an den Filter angeformt ist.

Die erste Sammelleitung kann mit einem die zu filtrierende Flüssigkeit enthaltenden Behältnis direkt verbunden sein. Es ist aber auch möglich, dass die Sammelleitung mit einem Konnektorsystem mit dem Behältnis verbunden ist. Derartige sterile Verbindungseinrichtungen sind allgemein bekannt.

In einer weiteren bevorzugten Ausführungsform ist der Filter ein Filter zur Entfernung von Leukozyten. Der Filter kann aber auch ein Filter zur Entfernung von anderen Blutkomponenten aus Vollblut oder anderen Komponenten aus anderen biologischen oder medizinischen Flüssigkeiten sein.

Im Folgenden wird ein Ausführungsbeispiel unter Bezugnahme auf die Zeichnungen näher erläutert.

Es zeigen:
- Figur 1: eine bevorzugte Ausführungsform eines Filtriersystems in schematischer Darstellung und
- Figur 2: eine Teilansicht des Filtriersystems in vergrösserter Darstellung.

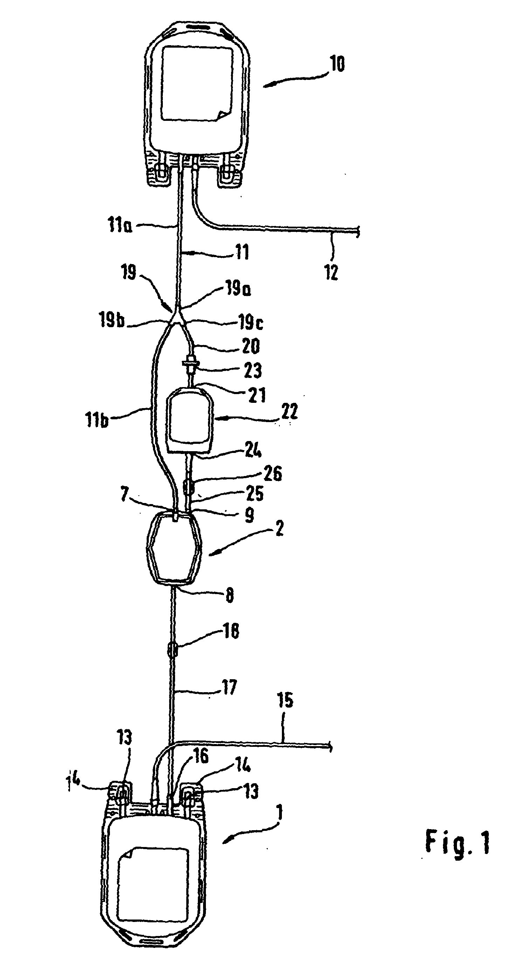

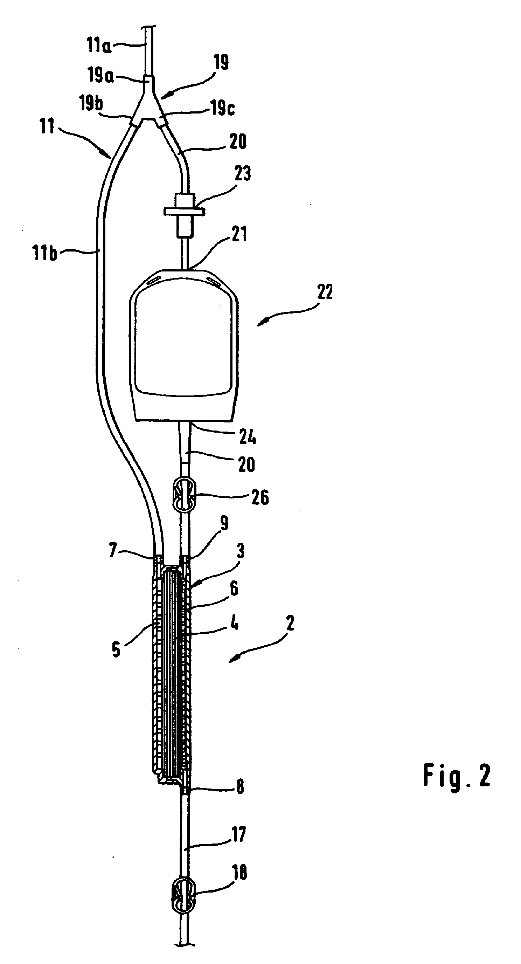

Figur 1 zeigt eine bevorzugte Ausführungsform des Filtriersystems in der Draufsicht. Das Filtriersystem umfasst ein Sammelbehältnis 1 zur Aufnahme der filtrierten Flüssigkeit, beispielseise eines leukozytenfreien Thrombozytenkonzentrats, und einen Filter 2 zur Entfernung einer Blutkomponente, beispielsweise einen Leukozytenfilter.
Die Seitenansicht des Filters 2 zeigt Figur 2 in vergrösserter Darstellung. Der Filter weist ein flaches Gehäuse 3 auf, das von einem Filtermaterial 4 in eine erste und zweite Filterkammer 5, 6 unterteilt ist. Die erste Filterkammer 5 weist an dem oberen Gehäuserand einen Einlass 7 und die zweite Filterkammer 6 an dem unteren Gehäuserand einen Auslass 8 und an dem oberen Gehäuserand einen Lufteinlass- bzw. Belüftungsanschluss 9 auf.

Die zu filtrierende Flüssigkeit, beispielsweise ein Thrombozytenkonzentrat, wird in einem Vorlagebehältnis 10, insbesondere in einem flexiblen Kunststoffbeutel bereitgestellt, der aus zwei an ihren Rändern miteinander verschweissten Kunststofffolien besteht und mit dem Filtriersystem verbunden ist. An dem Vorlagebehältnis 10 ist eine erste Sammelleitung 11 in Form einer flexiblen Schlauchleitung angeschlossen, die zu dem Einlass 7 des Filters 2 führt. Die Sammelleitung 11 ist mittels der bekannten Verbindungseinrichtungen steril mit dem Vorlagebehältnis verbunden. In Figur 1 ist neben der ersten Sammelleitung 11 noch eine weitere Schlauchleitung 12 angedeutet, die der Zufuhr von Blut oder einer Blutkomponente in das Vorlagebehältnis dienen kann.

Das Sammelbehältnis 1 ist ebenfalls ein flexibler Kunststoffbeutel. An dem oberen Rand des Kunststoffbeutels sind zwei mit durchstechbaren Membranen verschlossene Anschlussstutzen 13 angeschweisst, die jeweils mit aufreissbaren Laschen 14 steril verschlossen sind. Die Anschlussstutzen 13 können beipielsweise zur Entnahme eines leukozytenfreien Thrombozytenkonzentrats mittels geeigneter Spike-Konnektoren dienen. Darüber hinaus ist in Figur 1 eine weitere Schlauchleitung 15 angedeutet, die von dem Sammelbehältnis 1 abgeht. Diese Schlauchleitung kann der Entnahme einer Blutkomponente dienen.

Das Sammelbehältnis 1 weist einen Einlass 16 auf, der über eine zweite Sammelleitung 17 wieder in Form einer flexiblen Schlauchleitung mit dem Auslass 8 der zweiten Filterkammer 6 des Filters 2 verbunden ist. Zum Öffnen und Verschliessen der zweiten Sammelleitung 17 ist eine erste Schlauchklemme 18 an der Leitung vorgesehen.

Die erste Sammelleitung 11 weist einen ersten Abschnitt 11 a auf, der zu dem gemeinsamen Anschlussstück 19a eines Abzweigungselements 19 führt; während ein zweiter Abschnitt 11 b der ersten Sammelleitung 11 von dem einen Abzweigungsstück 19b des Abzweigungselements 19 abgeht und zu dem Einlass 7 der ersten Kammer 5 des Filters 2 führt. Von dem anderen Abzweigungsstück 19c des Abzweigungselements 19 geht eine erste Lufteinlass- bzw. -auslassleitung 20 ab, die zu einem ersten Luftein- bzw. -auslass 21 eines kollabierbaren Behältnisses 22 in Form eines flexiblen Kunststoffbeutels führt. Das Schlauchleitungsende ist mit dem oberen Rand des Kunststoffbeutels fest verschweisst. Vor dem Luftein- bzw. -auslass 21 des kollabierbaren Behältnisses 22 sitzt in der ersten Lufteinlass- bzw. -auslassleitung 20 ein Zwischenstück 23 mit einer hydrophoben, d. h. gasdurchlässigen, aber für Flüssigkeiten undurchlässigen Membran, die den Luftein- bzw. -auslass 21 verschliesst. Anstelle einer hydrophoben Membran kann das Zwischenstück aber auch ein Rückschlagventil enthalten, das in Richtung der ersten Sammelleitung für Fluid durchlässig, aber in Richtung des kollabierbaren Behältnisses undurchlässig ist.

Von einem zweiten Luftein- bzw. -auslass 24 des kollabierbaren Behältnisses 22 geht eine zweite Lufteinlass- bzw. -auslassleitung 25 ab, die zu dem Lufteinlass- bzw. Belüftungsanschluss 9 der zweiten Kammer 6 des Filters 2 führt. Das obere Ende der zweiten Lufteinlass- bzw. -auslassleitung 25 ist mit dem unteren Rand des kollabierbaren Behältnisses 22 fest verschweisst. Zum Öffnen und Verschliessen der zweiten Lufteinlass- bzw. -auslassleitung 25 ist eine zweite Schlauchklemme 26 vorgesehen, die auf der Schlauchleitung sitzt.

Das kollabierbare Behältnis 22 hat ein Volumen, das etwa dem Volumen der beiden Filterkammern 5, 6 des Filters 2 einschliesslich der angrenzenden Schlauchleitungen entspricht. Das Volumen des kollabierbaren Behältnisses sollte aber nicht kleiner sein als das Volumen des Filters zusammen mit den angrenzenden Leitungen, da ansonsten eine vollständige Entleerung des Filtriersystems nicht sichergestellt ist.

Nachfolgend wird das Verfahren zum Filtrieren von Flüssigkeiten unter Verwendung des Filtriersystems im Einzelnen beschrieben.

Zunächst wird die erste Schlauchklemme 18 der zweiten Sammelleitung 17 geschlossen und die zweite Schlauchklemme 26 an der zweiten Lufteinlass- bzw. - auslassleitung 25 geöffnet. Dabei wird vorausgesetzt, dass das Vorlagebehältnis 10 bereits an die erste Sammelleitung 11 des Filtriersystems angeschlossen ist.

Unter dem Einfluss der Schwerkraft strömt das Konzentrat aus dem Vorlagebehältnis 10 in den Filter 2 unter Verdrängung der eingeschlossenen Luft durch die zweite Lufteinlass- bzw. -auslassleitung 25 in das kollabierbare Behältnis 22. Das Volumen des kollabierbaren Behältnisses 22 ist derart bemessen, dass sich die erste Sammelleitung 11, die beiden Filterkammern 5, 6 des Filters 2 und der Abschnitt der zweiten Sammelleitung 17 stromauf der ersten Schlauchklemme 18 mit der zu filtrierenden Flüssigkeit füllen, wobei die verdrängte Luft in dem kollabierbaren Behältnis 22 verbleibt, d. h. das Behältnis 22 sich nicht mit Flüssigkeit füllt. Da der erste Luftein- bzw. -auslass 21 des Behältnisses 22 mit der hydrophoben Membran 23 oder dem Rückschlagventil 23 verschlossen ist, kann die in dem Behältnis eingeschlossene Luft nicht nach oben aufsteigen und entweichen. Andererseits kann aber Flüssigkeit über die erste Lufteinlass- bzw. -auslassleitung 20 nicht in das Behältnis 22 strömen.

Nachdem sich ein Gleichgewichtszustand eingestellt hat, wird die zweite Schlauchklemme 26 geschlossen und die erste Schlauchklemme 18 geöffnet. Die zu filtrierende Flüssigkeit strömt nun aus dem Vorlagebehältnis 10 über die erste und zweite Sammelleitung 11, 17 in das Sammelbehältnis 1. Wenn die Flüssigkeitsströmung zum Stillstand gekommen ist, wird die zweite Schlauchklemme 26 wieder geöffnet, so dass Luft aus dem kollabierbaren Behältnis 22 über die erste Lufteinlass- bzw. -auslassleitung 20 und den zweiten Abschnitt 11 b der ersten Sammelleitung 9 in den Filter 2 schlagartig angesaugt wird. Da die in dem kollabierbaren Behältnis 22 gesammelte Luft nunmehr über die erste Lufteinlass- oder -auslassleitung 20 und die erste Sammelleitung 20 in die erste Kammer 5 des Filters 2 strömt, entleert sich die erste Filterkammer 5 vollständig, ohne dass ein manueller Eingriff erforderlich ist. Anschließend entleert sich die zweite Filterkammer 6, während Luft aus dem Behältnis 22 über die zweite Lufteinlass- bzw. -auslassleitung 25 von oben in die zweite Filterkammer strömt und die Restflüssigkeit aus der zweiten Kammer über die zweite Sammelleitung 17 nach unten abfließt.

## Patentansprüche

1. Steriles System zum Filtrieren von Flüssigkeiten, insbesondere von Blut oder Blutkomponenten, mit
einem Filter (2), der ein Gehäuse (3) aufweist, das durch ein Filtermaterial (4) in zwei Kammern (5, 6) unterteilt ist, von denen die erste Kammer (5) mit einem Einlass (7) und die zweite Kammer (6) mit einem Auslass (8) versehen ist,
einer ersten mit dem Einlass (7) der ersten Filterkammer (5) verbundenen Sammelleitung (11) zum Zuführen der zu filtrierenden Flüssigkeit,
einem Sammelbehältnis (1), das einen Einlass (16) aufweist,
einer zweiten Sammelleitung (17), die den Auslass (8) der zweiten Filterkammer (6) mit dem Einlass (16) des Sammelbehältnisses (1) verbindet,
Mitteln (18) zum Öffnen bzw. Verschliessen der zweiten Sammelleitung (17),
einem kollabierbaren Behältnis (22), das eine erste Luftein- bzw. - auslassleitung (20), die mit der ersten Filterkammer (5) in Strömungsverbindung steht, und eine zweite Luftein- bzw. -auslassleitung (25) aufweist, die mit der zweiten Filterkammer (6) in Strömungsverbindung steht, und
Mitteln (26) zum Öffnen bzw. Verschliessen der zweiten Luftein- bzw. - auslassleitung (25),
**dadurch gekennzeichnet, dass** nur die erste Luftein- bzw. -auslassleitung (20) des kollabierbaren Behältnisses (22) mit einer hydrophoben Membran (23) oder einem Rückschlagventil (23), das einen Durchlass von Fluid nur in Richtung der ersten Filterkammer (5) erlaubt, verschlossen ist, wobei das Volumen des kollabierbaren Behältnisses (22) derart bemessen ist, dass sich die erste Sammelleitung (11), die beiden Filterkammern (5, 6) des Filters (2) und der Abschnitt der zweiten Sammelleitung (17) stromauf der Mittel (18) zum Öffnen bzw. Verschliessen der zweiten Sammelleitung (17) mit der zu filtrierenden Flüssigkeit füllen, wobei die verdrängte Luft in dem kollabierbaren Behältnis (22) verbleibt und das Behältnis (22) sich nicht mit Flüssigkeit füllt.

2. Steriles System nach Anspruch 1 , **dadurch gekennzeichnet, dass** die zweite Lufteinlass- bzw. -auslassleitung (25) an einer dem Auslass (8) der zweiten Filterkammer (6) des Filters (2) gegenüberliegenden Stelle in die zweite Filterkammer mündet.

3. Steriles System nach Anspruch 1 oder 2, **dadurch gekennzeichnet, dass** das kollabierbare Behältnis ein flexibler Kunststoffbeutel (22) ist.

4. Steriles System nach einem der Ansprüche 1 bis 3, **dadurch gekennzeichnet, dass** die erste Lufteinlass- bzw. -auslassleitung (20) von der ersten Sammelleitung (11) abzweigt.

5. Steriles System nach einem der Ansprüche 1 bis 4, **dadurch gekennzeichnet, dass** die Mittel zum Öffnen bzw. Verschliessen der zweiten Sammelleitung (11) und der zweiten Lufteinlass- bzw. -auslassleitung (25) auf den Leitungen sitzende Schlauchklemmen (18, 26) sind.

6. Steriles System nach einem der Ansprüche 1 bis 5, **dadurch gekennzeichnet, dass** der Filter ein Leukozytenfilter (2) ist.

7. Verfahren zum Filtrieren von Flüssigkeiten, insbesondere von Blut oder Blutkomponenten, mit einem sterilen System nach einem der Ansprüche 1 bis 6 mit folgenden Verfahrensschritten :
Schliessen der Mittel (18) zum Öffnen bzw. Verschliessen der zweiten Sammelleitung (17) und Öffnen der Mittel (26) zum Öffnen bzw. Verschliessen der zweiten Luftein- bzw. -auslassleitung (25),
Leiten der zu filtrierenden Flüssigkeit durch die erste Sammelleitung (11) über die erste Kammer (5) in die zweite Kammer (6) des Filters (2) unter Verdrängung der Luft in das kollabierbare Behältnis (22),
Verschliessen der Mittel (26) zum Öffnen bzw. Verschliessen der zweiten Luftein- bzw. -auslassleitung (25) und Öffnen der Mittel (18) zum Öffnen bzw. Verschliessen der zweiten Sammelleitung (17),
Leiten der Flüssigkeit durch die erste und zweite Sammelleitung (11, 17) in das Sammelbehältnis (1) und
Öffnen der Mittel (26) zum Öffnen bzw. Verschliessen der zweiten Luftein- bzw..-auslassleitung (25), so dass die in dem kollabierbaren Behältnis gesammelte Luft über die zweite Lufteinlass- bzw. -auslassleitung (25) in die zweite Filterkammer (6) zurückgeführt wird.

## Claims

1. A sterile system for filtering fluids, in particular blood or blood components, with
a filter (2), which has a housing (3) which is divided by a filter material (4) into two chambers (5, 6), whereof the first chamber (5) is provided with an inlet (7) and the second chamber (6) with an outlet (8),
a first collecting line (11) for feeding the fluid to be filtered, said collecting line being connected to the inlet (7) of the first filter chamber (5),
a collecting container (1) which has an inlet (16),
a second collecting line (17) which connects the outlet (8) of the second filter chamber (6) to the inlet (16) of the collecting container (1),
means (18) for the opening and closing of the second collecting line (17),
a collapsible container (22), which has a first air inlet and outlet line (20), which is in a flow connection with the first filter chamber (5), and a second air inlet and outlet line (25), which is in a flow connection with the second filter chamber (6), and
means (26) for the opening and closing of the second air inlet and outlet line (25),
**characterised in that** only the first air inlet and outlet line (20) of the collapsible container (22) is closed with a hydrophobic membrane (23) or a non-return valve (23) which permits a passage of fluid only in the direction of the first filter chamber (5), wherein the volume of the collapsible container (22) is dimensioned in such a way that the first collecting line (11), the two filter chambers (5, 6) of the filter (2) and the section of the second collecting line (17) upstream of the means (18) for the opening and closing of the second collecting line (17) are filled with the fluid to be filtered, wherein the displaced air remains in the collapsible container (22) and the container (22) is not filled with fluid.

2. The sterile system according to claim 1, **characterised in that** the second air inlet and outlet line (25) emerges into the second filter chamber at a point lying opposite the outlet (8) of the second filter chamber (6) of the filter (2).

3. The sterile system according to claim 1 or 2, **characterised in that** the collapsible container is a flexible plastic bag (22).

4. The sterile system according to any one of claims 1 to 3, **characterised in that** the first air inlet and outlet line (20) branches off from the first collecting line (11).

5. The sterile system according to any one of claims 1 to 4, **characterised in that** the means for the opening and closing of the second collecting line (11) and the second air inlet and outlet line (25) are on tube clamps (18, 26) sitting on the lines.

6. The sterile system according to any one of claims 1 to 5, **characterised in that** the filter is a leucocyte filter (2).

7. A method for filtering fluids, in particular blood or blood components, with a sterile system according to any one of claims 1 to 6 with the following process steps,
closing of the means (18) for the opening and closing of the second collecting line (17) and opening of the means (26) for the opening and closing of the second air inlet and outlet line (25),
conveying of the fluid to be filtered through the first collecting line (11) via the first chamber (5) into the second chamber (6) of the filter (2) with displacement of the air into the collapsible container (22),
closing of the means (26) for the opening and closing of the second air inlet and outlet line (25) and opening of the means (18) for the opening and closing of the second collecting line (17),
conveying of the fluid through the first and second collecting line (11, 17) into the collecting container (1) and
opening of the means (26) for the opening and closing of the second air inlet and outlet line (25), so that the air collected in the collapsible container is fed back via the second air inlet and outlet line (25) into the second filter chamber (6).

## Revendications

1. Système stérile pour la filtration de liquides, en particulier de sang ou de constituants du sang, comprenant :
• un filtre (2) qui présente un boîtier (3) qui est subdivisé en deux chambres (5, 6) par un matériau de filtration (4), chambres dont la première chambre (5) est dotée d'une entrée (7) et la deuxième chambre (6) d'une sortie (8),
• un premier conduit collecteur (11) raccordé à l'entrée (7) de la première chambre de filtration (5) et servant à fournir le liquide à filtrer,
• un récipient collecteur (1) qui présente une entrée (16),
• un deuxième conduit collecteur (17) qui relie la sortie (8) de la deuxième chambre de filtration (6) à l'entrée (16) du récipient collecteur (1),
• des moyens (18) permettant d'ouvrir ou de fermer le deuxième conduit collecteur (17),
• un récipient aplatissable (22) qui présente un premier conduit (20) d'entrée ou de sortie d'air qui communique, pour l'écoulement, avec la première chambre de filtration (5), et un deuxième conduit (25) d'entrée ou de sortie d'air qui communique, pour l'écoulement, avec la deuxième chambre de filtration (6), et
• des moyens (26) permettant d'ouvrir ou de fermer le deuxième conduit (25) d'entrée ou de sortie d'air,
**caractérisé en ce que** seulement le premier conduit (20) d'entrée ou de sortie d'air du récipient aplatissable (22) est fermé par une membrane hydrophobe (23) ou par un clapet antiretour (23) qui permet un passage du fluide seulement dans la direction de la première chambre de filtration (5), où le volume du récipient aplatissable (22) est dimensionné de manière telle que le premier conduit collecteur (11), les deux chambres de filtration (5, 6) du filtre (2) et la partie du deuxième conduit collecteur (17) placée en amont des moyens (18) permettant l'ouverture ou la fermeture du deuxième conduit collecteur (17) se remplissent du liquide à filtrer, où l'air chassé reste dans le récipient aplatissable (22), et le récipient (22) ne se remplit pas de liquide.

2. Système stérile selon la revendication 1, **caractérisé en ce que** le deuxième conduit (25) d'entrée ou de sortie d'air débouche dans la deuxième chambre de filtration, au niveau d'un emplacement placé à l'opposé de la sortie (8) de la deuxième chambre de filtration (6) du filtre (2).

3. Système stérile selon la revendication 1 ou 2, **caractérisé en ce que** le récipient aplatissable est une poche (22) en matière plastique flexible.

4. Système stérile selon l'une quelconque des revendications 1 à 3, **caractérisé en ce que** le premier conduit (20) d'entrée ou de sortie d'air bifurque par rapport au premier conduit collecteur (11).

5. Système stérile selon l'une quelconque des revendications 1 à 4, **caractérisé en ce que** les moyens permettant l'ouverture ou la fermeture du deuxième conduit collecteur (11) et du deuxième conduit (25) d'entrée ou de sortie d'air sont des colliers de serrage (18, 26) placés sur les conduits.

6. Système stérile selon l'une quelconque des revendications 1 à 5, **caractérisé en ce que** le filtre est un filtre à leucocytes (2).

7. Procédé de filtration de liquides, en particulier de sang ou de constituants du sang, comprenant un système stérile selon l'une quelconque des revendications 1 à 6, comportant les étapes de procédé qui suivent consistant :
• à fermer les moyens (18) permettant l'ouverture ou la fermeture du deuxième conduit collecteur (17), et à ouvrir les moyens (26) permettant l'ouverture ou la fermeture du deuxième conduit (25) d'entrée ou de sortie d'air,
• à diriger le liquide à filtrer à travers le premier conduit collecteur (11), dans la deuxième chambre (6) du filtre (2), via la première chambre (5), en chassant l'air contenu dans le récipient aplatissable (22),
• à fermer les moyens (26) permettant l'ouverture ou la fermeture du deuxième conduit (25) d'entrée ou de sortie d'air, et à ouvrir les moyens (18) permettant l'ouverture ou la fermeture du deuxième conduit collecteur (17),
• à diriger le liquide dans le récipient collecteur (1), à travers les premier et deuxième conduits collecteurs (11, 17), et
• à ouvrir les moyens (26) permettant l'ouverture ou la fermeture du deuxième conduit (25) d'entrée ou de sortie d'air, de sorte que l'air accumulé dans le récipient aplatissable soit redirigé dans la deuxième chambre de filtration (6), via le deuxième conduit (25) d'entrée ou de sortie d'air.
